# EUROPEAN PATENT APPLICATION

(11) **EP 4 085 847 A1**
(43) Date of publication of application: **09.11.2022**
(21) Application number: 22169931.7
(22) Date of filing: 26.04.2022
(51) Int. Cl.: A61B 17/04

(54) **KNOTLESS SUTURE ANCHOR SYSTEM WITH SUTURE MANAGEMENT STRUCTURE**

(30) Priority: 27.04.2021 US 202117241456
(71) Applicant: Medos International Sarl, 2400 Le Locle (CH)
(72) Inventor: GABRIEL, Stefan M., Mattapoisett, 02739 (US); SENGUN, Mehmet Ziya, Canton, 02021 (US); WHITTAKER, Gregory R., Stoneham, 02180 (US); CLEVELAND, Benjamin, Milford, 01757 (US); SHAINWALD, Mark, Raynham, 02767 (US); GUSTAFSON, Adam, Dighton, 02715 (US); GAMACHE, Daniel, Dedham, 02026 (US)
(74) Representative: Carpmaels & Ransford LLP

(57) **Abstract**

Methods and systems are provided for securing tissue to bone. A suture anchor system can include an inserter tool having a lumen and an inserter shaft with an anchor receiving portion that is proximal to a distal extension. The anchor receiving portion can have a drive feature and the distal extension can have a diameter less than the anchor receiving portion. An anchor can be disposed on the anchor receiving portion of the inserter tool, and can have a driven feature disposed in a lumen configured to engage the drive member of the anchor receiving portion and a bone-engaging feature disposed on an external surface. A suture seating member can be removably disposed on the distal extension, can be rotatable independent of the anchor, and can have a lumen with an opening on a distal end, where the distal end of the suture seating member has a suture seating surface.

## Description

### FIELD

The present disclosure relates generally to methods and devices for securing soft tissue to bone.

### BACKGROUND

Tearing of, or complete or partial detachment of ligaments, tendons and/or other soft tissues from their associated bones within the body are commonplace injuries. Such injuries can result from excessive stresses being placed on these tissues. By way of example, tissue tearing or detachment may occur as the result of an accident such as a fall, over-exertion during a work-related activity, or during the course of an athletic event. In the case of tearing or a partial or complete detachment of soft tissue from a bone, surgery is typically required to reattach the soft tissue (or a graft tissue) to the bone.

One method of repairing such a tear is to stitch it closed by passing a length of suture through the tissue and tying the suture. Suture can also be used in conjunction with one or more suture anchors to repair such tissue tears. Sutures can be fastened to suture anchors and to tissue using knots tied by the surgeon during a repair procedure, or using "knotless" devices and methods, where one or more anchors and one or more sutures can be connected and tensioned without the surgeon needing to tie knots during the surgery. Knotless anchoring is of particular utility for minimally invasive surgeries, such as endoscopic or arthroscopic repairs, where the surgeon remotely manipulates the suture at the surgical site using tools inserted through a small percutaneous incision, small diameter cannula, or an endoscopic tube, which can make the knottying process difficult and tedious.

It can be challenging to maintain the desired alignment and tension on the operative suture in the course of a surgical procedure to reattach soft tissue. Further, existing suture anchors used to insert the anchors into bone may have certain disadvantages that complicate their use and/or impose certain undesirable limits.

Accordingly, there is a need for improved devices, systems, and methods for attaching tissue to bone.

### SUMMARY

In one aspect, suture anchor system includes a cannulated inserter tool having a lumen extending therethrough and an elongate inserter shaft with an anchor receiving portion that is proximal to a distal extension. The anchor receiving portion has at least one drive feature formed thereon and the distal extension has a diameter that is less than a diameter of the anchor receiving portion. A cannulated suture anchor is removably disposed on the anchor receiving portion of the inserter tool. The suture anchor has at least one bone-engaging feature disposed on an external surface thereof. A cannulated suture seating member is removably disposed on the distal extension in a clearance fit. The suture seating member being rotationally independent of the suture anchor and having a lumen extending therethrough with an opening on a distal portion thereof, where the distal end of the suture seating member has a suture seating surface.

The suture anchor system can have various configurations. For example, at least one stay suture loop can extend through the lumen and the opening of the suture seating member.

The suture seating member can have various configurations. For example, the suture seating surface can be at least one of a flat surface and a contoured surface. The contoured surface can be a generally concave surface. The suture seating member can further include a collapsible section, and the suture seating surface can be arranged on the distal end of the collapsible section. The suture seating member can include a pair of opposed suture seating features arranged on opposed side surfaces of the suture seating member, the suture seating features being configured to maintain a desired orientation of one or more operative suture strands. The suture seating member can be spaced a distance from the suture anchor along the inserter shaft. The suture seating surface can be configured to maintain one or more operative sutures in a predetermined orientation.

The cannulated inserter tool can have various configurations. For example, a length of the anchor receiving portion can be greater than or equal to a length of the suture anchor. The anchor receiving portion can have a non-cylindrical cross-sectional shape. The anchor receiving portion can have a hexagonal cross-sectional shape. A distal end of the anchor receiving portion can include a shoulder that is adjacent to a proximal end of the suture seating member.

The suture anchor can have various configurations. For example, the suture anchor can include at least one driven feature disposed in a lumen thereof that is configured to engage the drive member of the anchor receiving portion. The lumen of the suture anchor can have a cross-sectional shape that is complementary to a cross-sectional shape of the anchor receiving portion.

A method for attaching soft tissue to bone is described which can include securing one or more operative sutures to a soft tissue to be reattached to bone. Free suture limbs of one or more operative sutures are passed through at least one stay suture loop formed from at least one stay suture, with the at least one stay suture loop extending from an opening on a distal end of a cannulated suture seating member, and the suture seating member being positioned distally to a suture anchor removably mounted on a cannulated inserter. The at least one stay suture is tensioned to close the at least one stay suture loop such that the one or more operative sutures are trapped between a suture seating surface arranged on the suture seating member and the at least one stay suture loop. The one or more operative sutures are placed within a bone hole adjacent the soft tissue to be reattached to bone by inserting into the hole the inserter having the anchor and suture seating member mounted thereon. A desired amount of tension is applied to the free suture limbs while the one or more operative sutures are positioned within the hole. The suture anchor is implanted into the bone hole to engage bone while maintaining the desired tension on the one or more operative sutures to bring the soft tissue into a desired engagement with bone while maintaining a rotational position of the suture seating member and substantially maintaining a position of the free suture limbs of the one or more operative sutures relative to the suture seating member. The at least one stay suture and inserter are then removed.

The method can have various configurations. For example, implanting the suture anchor can comprise rotating the suture anchor. Following removal of the inserter and the at least one stay suture, the one or more operative sutures can be knotlessly secured between the suture anchor and the bone hole. Following removal of the inserter and the at least one stay suture, the suture anchor can compress the suture seating member against a bottom of the bone hole and the suture seating member can compress against the bottom of the bone hole a portion of the one or more operative sutures distal to the suture seating member.

The cannulated suture anchor can have various configurations. For example, cannulated suture anchor can include at least one bone-engaging feature disposed on an external surface thereof. The at least one bone-engaging feature can be one of a thread and bone engaging barbs.

The cannulated suture seating member can have various configurations. For example, the suture seating member can be not rotated when the suture anchor is rotated.

### BRIEF DESCRIPTION OF THE DRAWINGS

The present disclosure will be more fully understood from the following detailed description taken in conjunction with the accompanying drawings, in which:
FIG. 1 is a cross-sectional view of a suture anchor system;
FIG. 2 is a cross-sectional view of a suture seating member included in the suture anchor system of FIG. 1;
FIGS. 3A and 3B are front and perspective views, respectively, of another suture seating member in an uncompressed condition;
FIGS. 3C and 3D are front and perspective views, respectively, of the suture seating member of FIGS. 3A and 3B in a compressed condition;
FIG. 4 is a cross-sectional view of another suture seating member;
FIG. 5 is a perspective view of another suture seating member;
FIG. 6 is a perspective view of another suture seating member;
FIG. 7 is a perspective view of another suture seating member;
FIG. 8A is a cross-sectional view of another suture seating member;
FIG. 8B is a cross-sectional view of the suture seating member of FIG. 8A in a deployed position;
FIGS. 9A and 9B are perspective views of another suture anchor system including the suture seating member of FIG. 6; and
FIGS. 9C-9E schematically illustrate steps in a method of using the surgical anchor system of FIGS. 9A-9B to reattach soft tissue to bone.

### DETAILED DESCRIPTION

Those skilled in the art will understand that the devices and methods specifically described herein and illustrated in the accompanying drawings are non-limiting and that the scope of the present invention is defined solely by the claims.

Further, in the present disclosure, like-named components generally have similar features, and thus each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used. In addition, the terms "about" and "substantially" are defined as ranges based on manufacturing variations and variations over temperature and other parameters.

Successful soft tissue repair surgery, such as rotator cuff repair, requires that the suture anchor be securely engaged within bone to avoid migration and/or dislodgement, and that the operative suture attached to the soft tissue and maintained against the bone by the suture anchor resist migration when subjected to loads. The most common mode of failure is due to suture migration or slippage, which occurs when the load applied to the operative suture exceeds the force resisting such slippage or migration. This resisting force results from the suture anchor compressing the operative suture against the bone in which the suture anchor is implanted. Suture migration occurs at lower loads when the sutures themselves are misaligned and overlap within the bone hole. This can be particularly disadvantageous when an area of the bone adjacent to one of more strands of suture is softer and/or less healthy. The suture anchor systems described herein provide anchor implantation system designs optimized to maintain alignment of the operative suture (e.g., by maintaining the suture in a desired positon or with desired spacing between operative suture strands) during and following insertion of the suture anchor to avoid suture migration and overlap, while maintaining secure engagement of the anchor within bone.

A suture anchor system is provided that includes a suture seating member that enables the strands of the operative suture to be separated and maintained in a desired position around the anchor with sufficient spacing to prevent an undue degree of suture overlap while enabling the anchor to be inserted into the bone to secure soft tissue to the bone using the operative suture. During the course of a surgical procedure utilizing the suture anchor systems described herein, which is typically conducted arthroscopically, a suture anchor is removably disposed on an elongate inserter shaft of an inserter tool for delivery to its implantation site where it will engage operative sutures to maintain the operative suture in tension and thus reattach soft tissue to bone. The suture anchor system also includes a suture seating member removably mounted on the inserter device distal to the suture anchor. The suture seating member is mounted in such a way that it is rotationally independent of the suture anchor and the inserter tool. As explained in more detail below, the suture seating member includes at its distal end a suture seating surface that assists in maintaining the suture strands in a desired position and/or with a suitable degree of separation.

The suture seating surface of the suture seating member can be any sort of distally oriented surface that can assist in maintaining separation, spacing, or position of the operative suture strands. The suture seating surface of the suture seating member can be a flat surface, a contoured surface, and/or other surface shape arranged at the distal-facing surface of the suture seating member. In this way, the operative suture can be captured by the suture seating surface while the suture anchor is being inserted to maintain the operative suture in proper alignment and/or with sufficient spacing between strands to prevent any undesired repositioning or overlap of the suture strands as the anchor is rotated into or otherwise implanted into bone.

The suture seating member can be attached to the inserter tool and/or the suture anchor in such a way that the suture seating member is independently rotatable relative to the suture anchor, forming an implantable suture anchor construct. That is, rotation of the suture anchor will not result in rotation of the suture seating member. Independent rotation of the suture seating member can be achieved in a variety of ways. For example, the suture seating member need not be attached to the suture anchor. In this way, any rotation of the suture anchor will not result in rotation of the suture seating member, thus maintaining the desired position of the suture strands as established by the suture seating surface.

A person skilled in the art will appreciate that the suture anchor systems described herein can be used in a variety of surgical techniques to secure and/or reattach soft tissue to bone. The surgical procedure can be conducted as an open surgical procedure or as a minimally invasive surgical procedure, such as an arthroscopic procedure. Following preparation of the patient, an appropriate incision is made to access the surgical site. One or more holes are formed in bone as required by the particular surgical procedure, in proximity to the tissue repair site, to receive the one or more anchors to be used to anchor the soft tissue. One or more strands of operative suture are then passed through the detached tissue and then passed through a stay suture or positioned adjacent to the one or more suture anchors to be used in the procedure. The one or more suture anchors are then implanted into the corresponding bone hole(s) using an appropriate inserter tool as the operative sutures are appropriately tensioned to bring the detached tissue in proximity to bone. In so doing, the operative suture(s) are compressed within the bone hole between the anchor and the wall that defines the bone hole. Excess suture is then removed, and the incision is closed.

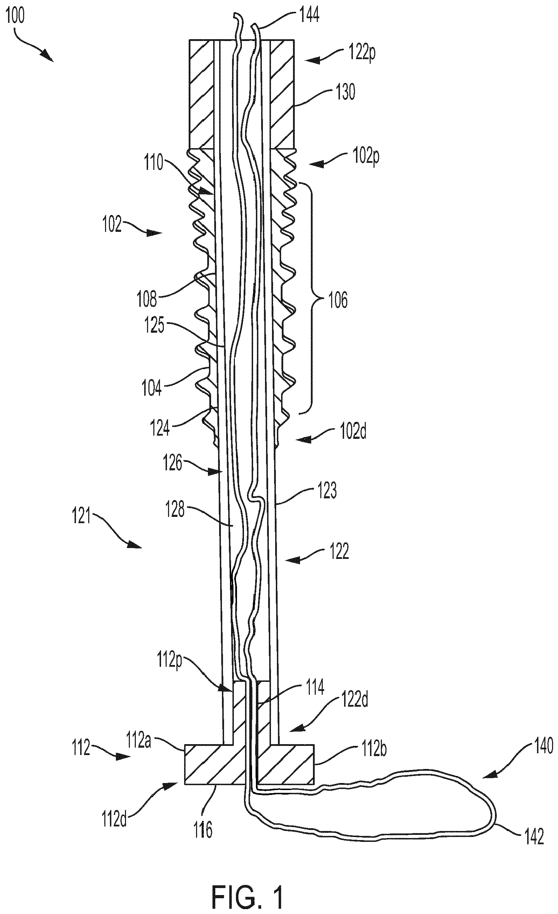

FIGS. 1-2 illustrate a suture anchor system 100 used to help maintain a desired position of suture strands during insertion of a knotless suture anchor. The suture anchor system 100 generally includes a cannulated inserter tool 121 on which a cannulated suture anchor 102 is removably mounted, and a cannulated suture seating member 112 that is removably mounted at the distal end of the inserter tool 121. The suture anchor 102 and the suture seating member 112 form an anchor construct that is implanted into a bone hole to secure soft tissue to bone. The inserter tool 121 aids in the alignment and insertion of the anchor construct, and is removed once the anchor construct is fully inserted into a bone hole.

The cannulated suture anchor 102 is configured to secure operative suture strands within a bone hole between the external surface of the anchor and the bone itself to reattach soft tissue to bone. The suture anchor 102 generally includes a proximal end 102p, a distal end 102d, and an external surface 104 with a series of bone-engaging features 106. Each of the plurality of longitudinally spaced bone-engaging features 106 can be formed at least partially circumferentially on the external surface 104 over at least a portion of a length of the suture anchor 102. Additionally, the suture anchor 102 can include a lumen 108 extending through the suture anchor 102 from the proximal end 102p to the distal end 102d, with the lumen 108 being configured to removably secure the anchor 102 to an inserter device, as described in detail below.

In suture anchor systems where the suture anchor 102 includes threaded bone-engaging features 106, the internal surface of the lumen 108 includes a driven feature 110 formed into the lumen. While the driven feature 110 can take a variety of forms that will be understood by a person skilled in the art, in the system illustrated in FIG. 1, the driven feature 110 is a female hexagonal cross-sectional shape formed by the lumen 108 of the anchor 102. Alternative designs for the driven feature 110 can include but are not limited to a variety of other shapes which have the ability to transfer rotational movement to the anchor, including an oval shape, a square shape, a pentagonal shape, etc. The driven feature 110 is configured to enable rotational movement to be imparted to the anchor 102 in order to rotate the suture anchor 102, which causes the threads of the bone-engaging features 106 to cut into a bone hole during a surgical procedure using the suture anchor system 100.

In order to ensure a suitable retention force of the anchor 102, the series of bone-engaging features 106 can be disposed over at least a portion of, and generally a majority of, a length of the external surface 104 of the anchor 102. Additionally, the series of bone-engaging features 106 can collectively encompass about 360° of a circumference of the anchor 102. While the bone-engaging features 106 can take a variety of forms that will be understood by a person skilled in the art, in the system illustrated in FIG. 1, the bone-engaging features 106 are in the form of a helical thread. As shown in the illustrated system by way of example, the shape of the thread form may vary along the length of the suture anchor 102. Alternative designs for the bone-engaging features 106 can include but are not limited to a plurality of barbs, etc.

The inserter device is used to apply a force (e.g., a rotational force) to the suture anchor to drive the anchor 102 into bone. In this system, the inserter tool 121 can be configured to rotate the suture anchor 102 into a bone hole. The inserter tool 121 generally includes an elongate inserter shaft 122 having a distal end 122d and a proximal end 122p with a lumen 128 extending therebetween. In some systems, the lumen 128 has a smooth inner surface to help prevent rotational motion from being transferred to a suture seating member during rotation of the inserter tool 121.

The inserter shaft 122 includes an anchor receiving portion 125 arranged somewhat proximal to the distal end 122d. The anchor 102 can be configured to be removably arranged on the inserter shaft 122 at the anchor receiving portion 125, with the inserter shaft 122 disposed within the lumen 108 of the anchor 102. The inserter shaft 122 can have a drive feature 126 arranged on an external surface 124 at the anchor receiving portion 125 of the inserter shaft 122. The drive feature 126 has a shape that is complimentary to that of the driven feature 110 of the suture anchor 102 in order to transfer rotation motion from the inserter shaft 122 to the suture anchor 102. While the drive feature 126 can take a variety of forms that will be understood by a person skilled in the art, in the system illustrated in FIG. 1, the drive feature 126 is a male hexagonal cross-sectional shape formed by the external surface 124 of the anchor receiving portion 125. Alternative designs for the drive feature 126 can include but are not limited to a variety of other shapes which have the ability to transfer rotational movement to the anchor, including an oval shape, a square shape, a pentagonal shape, etc.

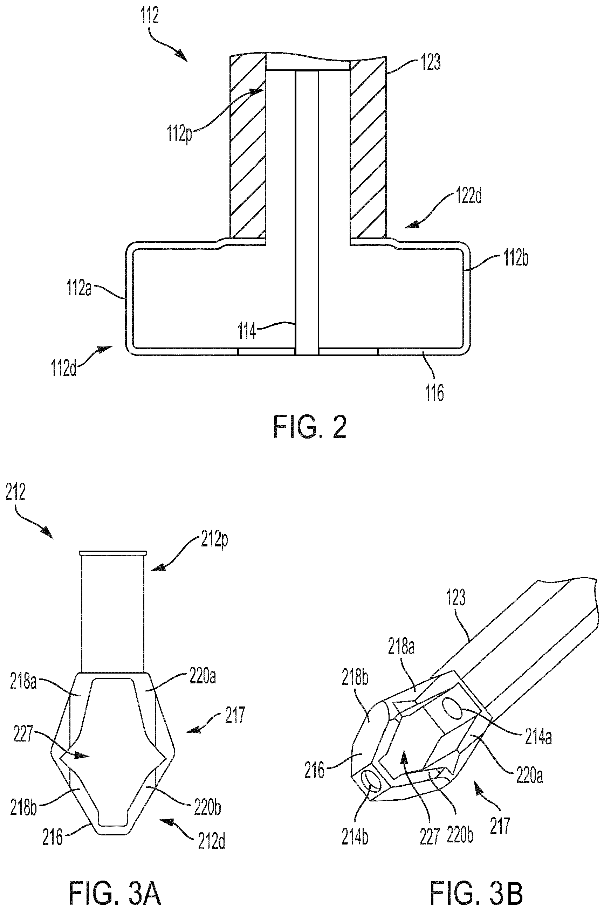

In addition to the anchor receiving portion 125, the inserter shaft 122 can include a distal extension 123 at the distal end 122d that extends distally beyond the suture anchor 102. The distal extension 123 is configured to removably seat the suture seating member 112 distal to the suture anchor 102. In one example, and as illustrated in FIGS. 1 and 2, the suture seating member 112 is configured to be mountable on the distal extension 123, such as with a portion of the suture seating member 112 being arranged within the lumen 128. The distal extension 123 can have a diameter that is less than the diameter of the anchor receiving portion 125.

The inserter shaft can have various configurations. For example, the inserter shaft 122 can have a length that makes it suitable for arthroscopic procedures. That is, the length of inserter shaft 122 is sufficient to have a distal end that seats the suture anchor 102 and the suture seating member 112 that form the anchor construct such that the anchor construct can access a hole drilled in bone while the proximal end 122p of the inserter shaft extends out of the body and can be manipulated by a surgeon. The distal end 122d of the inserter shaft 122 should be sufficient to seat the suture seating member 112 at a distal most end thereof and to seat the suture anchor 102 proximal to the suture seating member 112. Although the suture seating member 112 is shown to be spaced apart from the suture anchor 102 in FIG. 1, it is understood that the suture seating member 112 can be immediately adjacent to the suture anchor 102. As shown, the inserter shaft 122 can also include a handle 130 arranged at the proximal end 122p of the inserter shaft 122, which is configured to transfer force, such as a rotational motion, to the inserter shaft 122 and thus to the suture anchor 102.

The cannulated suture seating member 112 is a cannulated member that helps maintain the position and alignment of operative sutures, as well as a suitable amount of tension on the operative sutures, during implantation, such as by rotational insertion, of the suture anchor 102 into a bone hole.

While the suture seating member 112 can take a variety of forms, in the system illustrated in FIGS. 1 and 2 it is substantially in the shape of an inverted "T", having a central stem with radial extensions 112a, 112b arranged on the distal end 112d. The suture seating member 112 can be cannulated, having a lumen 114 extending completely therethrough from the proximal end 112p to the distal end 112d to facilitate passage of a stay suture therethrough. The radial extensions 112a, 112b extend outward perpendicular to the suture seating member 112, and are configured to extend to a length that is sufficient to allow the extensions 112a, 112b can contact the edges of a bone hole when inserted into a bone hole. The engagement of the extensions 112a, 112b with the boundaries of a bone hole when implanted therein serves to aid in the alignment of operative sutures passing over the suture seating member 112 since the operative sutures are prevented from changing position by passing around the side of the extensions 112a, 112b.

With the extensions 112a, 112b extending radially outward, a distal-facing suture seating surface 116 is formed by the extensions 112a, 112b. In this system, since the extensions 112a, 112b are perpendicular to the central stem of the suture seating member 112, the suture seating surface is a substantially flat surface. Due to the arrangement of the suture seating surface 116, operative sutures can extend distally towards the distal end 112d along a front side of the suture seating member 112, pass along and contact the suture seating surface 116, and then extend proximally back towards the suture anchor 102 along the back side of the suture seating member 112. In this way, operative sutures can be tensioned during insertion of the suture anchor 102, while the suture seating surface 116 maintains the operative sutures in a desired position on the suture seating member 112.

As mentioned above, the suture seating member 112 can be arranged relative to the suture anchor 102 in such a way that the suture seating member 112 is independently rotatable relative to the suture anchor 102. Independent rotation of the suture seating member 112 can be achieved in a variety of ways. In the illustrated system of FIG. 1, a portion of the suture seating member 112 is arranged within the lumen 128 at the distal end 122d of the inserter shaft 122, and the proximal end 112p of the suture seating member 112 is spaced apart from the distal end 112d of the suture anchor 102. The internal surface of the lumen 128 can be a smooth surface, with the inner diameter of the lumen 128 being larger than the outer diameter of the central stem portion of the suture seating member 112 that is seated within the lumen 128 so as not to provide an interference fit. This arrangement allows the inserter shaft 122 to transfer rotational movement to the suture anchor 102 without rotating the suture seating member 112. Therefore, the suture anchor 102 can be threaded into a bone hole while the suture seating member 112 does not rotate, keeping operative sutures in their intended positions when inserted into a bone hole.

In addition to the suture seating surface of the suture seating member 112, the suture anchor system 100 can further include a stay suture to aid in capturing and then aligning the operative sutures during insertion of the anchor construct. The stay suture 140 includes a loop 142 and free suture limbs 144. In some system, the stay suture 140 is removably disposed in the lumen 128 of the inserter shaft 122 such that a loop 142 of suture extends from the distal end 122d of the inserter shaft 122 and the two free suture limbs 144 extend from a proximal end 122p of the inserter shaft 122. The loop 142 is configured to be selectively tightened around operative sutures passing through the loop by tensioning one or both of the free suture limbs 144.

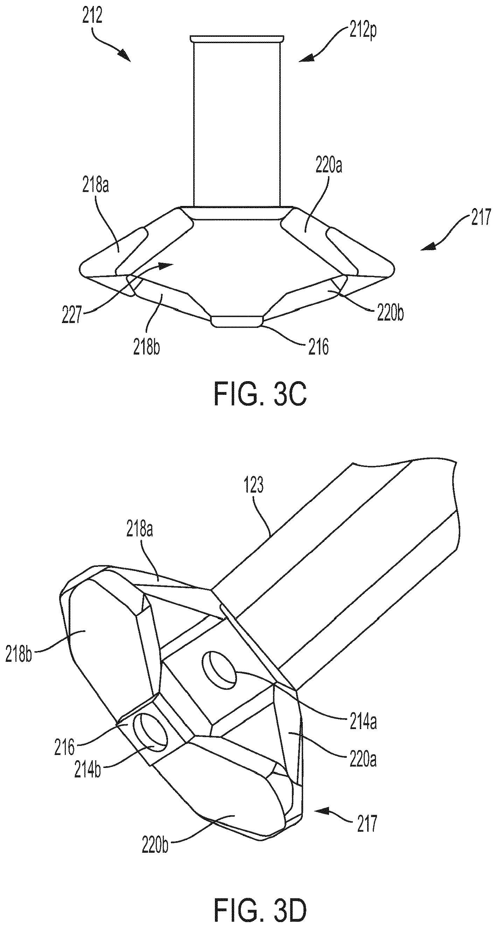

FIGS. 1 and 2 illustrate one suture seating member. However, the suture seating member can take a variety of forms. By way of non-limiting example, FIGS. 3-8B illustrate additional suture seating members that can be used with the suture anchor system 100. In each instance the suture seating member is cannulated and helps maintain the position and alignment of operative sutures, as well as a suitable amount of tension on the operative sutures, during rotational insertion of the suture anchor 102 into a bone hole.

Similar to the suture seating member 112, in the suture seating member illustrated in FIGS. 3A-3D, the suture seating member 212 has a central stem and a collapsible section extending distally therefrom that is substantially in the shape of a diamond in an uncompressed condition, and substantially in the shape of an inverted "T" in a compressed condition. The suture seating member 212 can be cannulated, having a lumen 214a extending completely therethrough the central stem from a proximal end 212p to a gap 227, and a lumen 214b extending completely therethrough from the gap 227 to a distal end 212d to facilitate passage of a stay suture therethrough.

As stated above, the collapsible extension section 217 is configured to be oriented in both uncompressed and compressed conditions. The suture seating member 212 is substantially in the shape of diamond when in the uncompressed condition, as shown in FIGS. 3A-3B. The suture seating member 212 has radial extensions 218a, 218b, 220a, 220b arranged on the distal end 212d, forming the collapsible section 217. The radial extensions 218a, 220a extend radially outward relative to the central stem of suture seating member 212. The radial extensions 218a, 220a transition to radial extensions 218b, 220b, which extend radially inward from radial extensions 218a, 220a towards a longitudinal axis of the suture seating member 212. The radial extensions 218b, 220b are configured to extend over a length that is sufficient to allow them to meet at gap 214b, forming the diamond shape of the collapsible section 217 in the uncompressed condition.

With the extensions 218b, 220b extending radially inward, a distal-facing suture seating surface 216 is formed by the extensions 218b, 220b. In this system, since the collapsible section 217 is in an uncompressed condition, the suture seating surface 216 is a substantially slanted surface.

When a compressive force is applied to the collapsible section 217, the extensions 218a, 220a pivot towards extensions 218b, 220b, thus reducing the gap 227, as illustrated in FIGS. 3C-3D, such that gap 227 of the suture seating member 212 is substantially in the shape of a broader diamond when compared to the uncompressed gap 227. In this system, since the collapsible section 217 is in a compressed condition, the suture seating surface 216 is a substantially flatter surface than in the uncompressed condition. Additionally, in a compressed condition, the extensions 218a, 220b are configured to extend to a width that is sufficient to allow the extensions 218a, 220a to contact the edges of a bone hole when the suture seating member 212.

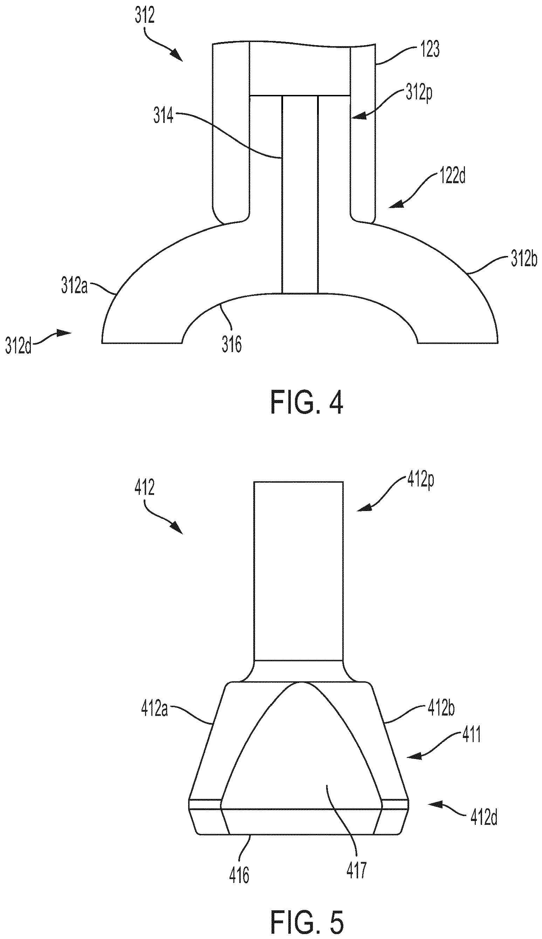

Similar to the suture seating member 112, in the system illustrated in FIG. 4, the suture seating member 312 has a central stem and curved radial extensions, and is substantially in the shape of an inverted "Y". The suture seating member 312 can be cannulated, having a lumen 314 extending completely therethrough the central stem from a proximal end 312p to a distal end 312d to facilitate passage of a stay suture therethrough. As stated above, the suture seating member 312 has curved radial extensions 312a, 312b arranged on the distal end 312d. The curved radial extensions 312a, 312b extend radially outward relative to the central stem of suture seating member 312 by a distance large enough such that the extensions 312a, 312b can contact the edges of a bone hole when inserted into a bone hole.

With the curved extensions 312a, 312b extending radially outward, a concave distal-facing suture seating surface 316 is formed by the extensions 312a, 312b. In this system, the suture seating surface 316 is a substantially curved surface, which helps prevent the operative sutures from disengaging with the suture seating surface 316 once tensioned.

Similar to the suture seating member 112, in the system illustrated in FIG. 5, the suture seating member 412 has a central stem and a body 411 that is substantially in the shape of a trapezoid. The suture seating member 412 can be cannulated, having a lumen (not shown) extending completely through the central stem from a proximal end 412p to a distal end 412d to facilitate passage of a stay suture therethrough. As stated above, the suture seating member 412 has a body 411 defined by opposed sidewalls 412a, 412b that are adjacent to opposed side suture seating surfaces 417 (only one of which is shown in FIG. 5). The sidewalls 412a, 412b extend diagonally outward from the central stem, and are configured to extend to a length that is sufficient to allow the sidewalls 412a, 412b to contact the edges of a bone hole when inserted into a bone hole.

With the sidewalls 412a, 412b and side suture seating surfaces 417 extending radially outward, a distal-facing suture seating surface 416 is formed by the body 411. In this system, the suture seating surface 416 is a flat surface, however one skilled in the art will appreciate that it can have other shapes, including concave. As shown in FIG. 5, the side suture seating surfaces 417 can be slightly concave surfaces that extend from the central stem to the suture seating surface 416. When implanted, the operative sutures contact the opposed side suture seating surfaces 417 and the suture seating surface 416.

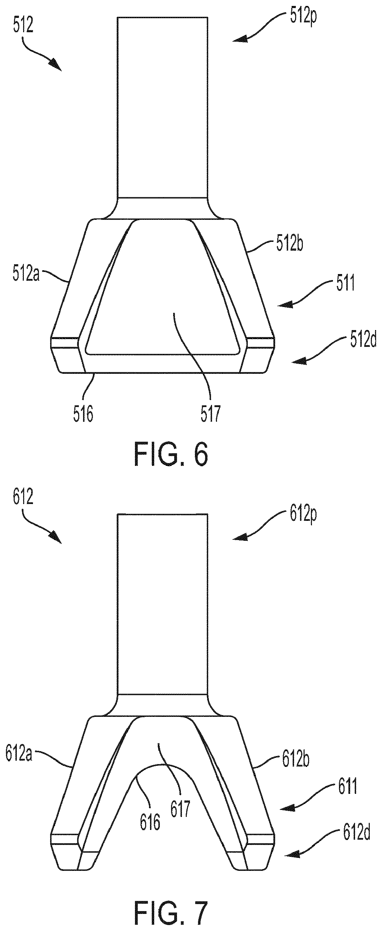

Similar to the suture seating member 412, in the system illustrated in FIG. 6, the suture seating member 512 has a central stem and a body 511 that is substantially in the shape of a trapezoid. The suture seating member 512 can be cannulated, having a lumen (not shown) extending completely through the central stem from a proximal end 512p to a distal end 512d to facilitate passage of a stay suture therethrough. As stated above, the suture seating member 512 has a body 511 that is defined by opposed sidewalls 512a, 512b that are adjacent to opposed side suture seating surfaces 517 (only one of which is shown in FIG. 6). The sidewalls 512a, 512b extend diagonally outward relative to the suture seating member 512, and are configured to extend to a length that is sufficient to allow the sidewalls 512a, 512b to contact the edges of a bone hole when inserted into a bone hole.

With the sidewalls 512a, 512b and side suture seating surfaces 517 extending radially outward, a distal-facing suture seating surface 516 is formed by the body 511. In this system, the suture seating surface 516 is a flat surface, however one skilled in the art will appreciate that it can have other shapes, including concave. As shown in FIG. 6, the side suture seating surfaces 517 can be concave surfaces that extend from the central stem to the suture seating surface 516. When implanted, the operative sutures contact the opposed side suture seating surfaces 517 and the suture seating surface 516.

FIG. 7 illustrates another suture seating member, in which the suture seating member 612 has a central stem and a body 611 that is substantially in the shape of an inverted "Y". The suture seating member 612 can be cannulated, having a lumen (not shown) extending completely through the central stem from a proximal end 612p to a distal end 612d to facilitate passage of a stay suture therethrough. The suture seating member 612 has a body 611 including opposed extensions 612a, 612b that define sidewalls of the body 611. The extensions 612a, 612b extend diagonally outward from the central stem and are configured to extend to a dimension that is sufficient to allow the extensions 612a, 612b to contact the edges of a bone hole when inserted into a bone hole.

A curved distal-facing suture seating surface 616 is formed in a gap between the extensions 612a, 612b, and in the illustrated system the suture seating surface 616 is a curved surface. Additionally, a pair of opposed concave side suture seating surfaces 617 are arranged on the side of the body 611 adjacent to extensions 612a, 612b. Thus, opposed surfaces 617 and suture seating surface 616 cooperate to form the surfaces that seat the operative suture, acting to maintain the position of the operative sutures and to prevent them from disengaging with the suture seating member 612.

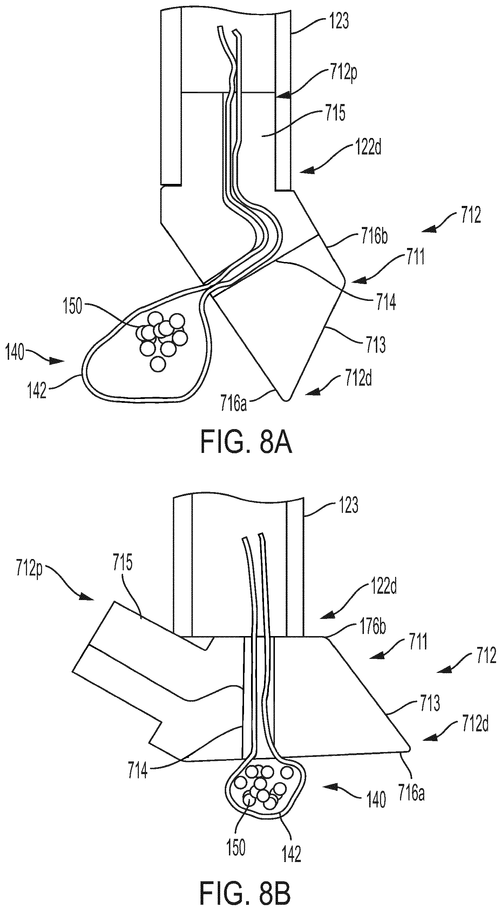

In some examples of a suture seating member, such as illustrated in FIGS. 8A-8B, the suture seating member can be rotated after insertion into the bone hole from an initial, delivery orientation (FIG. 8A) to a deployed orientation (FIG. 8B) to further secure the suture seating member within the bone hole. As shown in FIGS. 8A and 8B, suture seating member 712 has a central stem and a body 711, and is substantially in the shape of a trapezoid. The suture seating member 712 can be cannulated, having a lumen 714 extending substantially longitudinally through the central stem and then transversely to surface 716a. Further, a channel 715 extends through the body 711 from surface 716a to surface 716b. The channel 715 is formed such that it is in communication with the portion of the lumen 714 that turns towards surface 716a. Lumen 714 and channel 715 are configured to facilitate passage of a stay suture therethrough, as explained below. The suture seating member 712 has a body 711 including sidewalls 713, 716a, and 716b arranged on the edges of the body 611. The sidewalls 713, 716a extend diagonally inward relative to the suture seating member 712 to form a point at the distal end 712d. Additionally, the sidewall 716a is configured to extend to a length that is sufficient to allow the sidewall 716 to contact the edges of a bone hole when inserted into a bone hole and then rotated to its deployed orientation.

FIG. 8B illustrates the suture seating member 712 after having been rotated to its deployed orientation such that a distal-facing suture seating surface is formed by the sidewall 716a. While FIGS. 8A and 8B show surface 716a to be a flat surface, a person skilled in the art will appreciate that it can take other shapes as well, including concave. Transitioning the suture seating member 712 from it delivery orientation to its deployed orientation can be effected by dislodging the central stem from the distal extension 123, and then pushing it into a rotated position, such as by the distal extension 123. As the suture seating member 712 is being rotated, the stay suture 140 is dislodged from the lumen 715 and aligns axially within channel 714. The design of the suture seating member 712 enables it to be installed into a bone hole with ease due to the tapered design of the sidewalls 713, 716a, and 716b, and then rotated to be securely engaged within the bone hole.

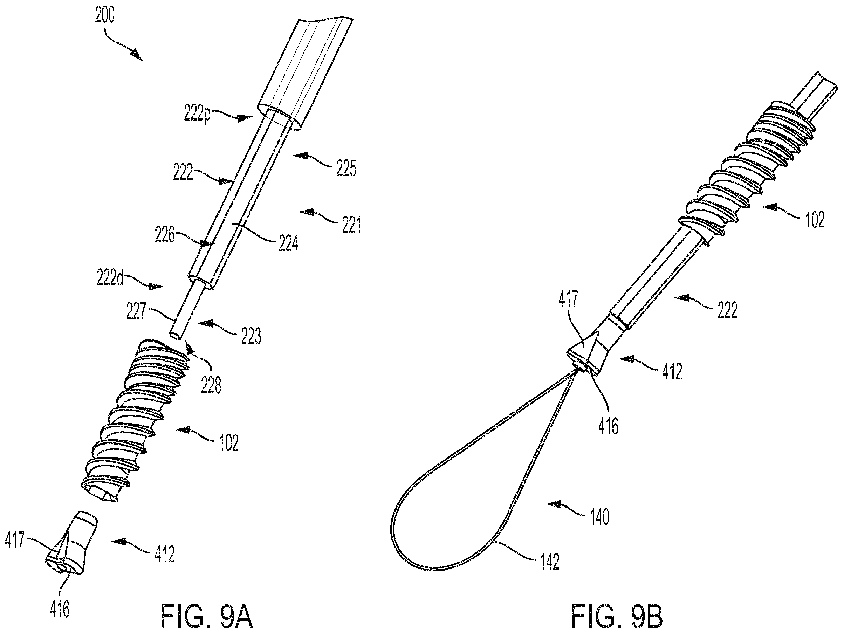

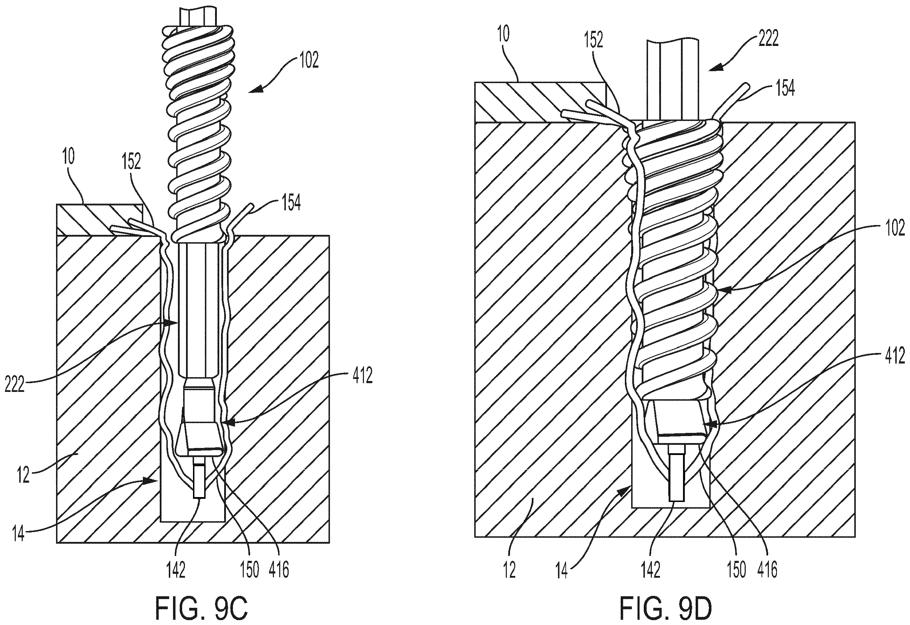

FIGS. 9A-9B illustrate another surgical anchor system including the suture seating member of FIG. 6, and FIGS. 9C-9E sequentially illustrate a procedure in which an anchor construct of the type described herein secures soft tissue 10 to bone 12 while the suture seating surface promotes positioning of and/or prevents overlapping of the operative suture.

As illustrated in FIGS. 9A and 9B, suture anchor 102 is mounted on inserter shaft 222 and the distal extension 223 of the inserter shaft seats the suture seating member 412 by passing into the lumen of the suture seating member. With the suture anchor 102 and the suture seating member 412 removably mounted on the inserter shaft 222 as so described, a capture suture loop 142 passes through a lumen that extends through the inserter shaft.

The entire assembly shown in FIG. 9B can be fully assembled, in sterile packaging, prior to the beginning of the procedure. To begin the procedure the patient is prepared in the usual manner and the surgical site is accessed in an appropriate manner, either by an open surgical technique or a minimally invasive technique, such as arthroscopically. A hole is formed in bone in proximity to the soft tissue 10 that is being secured to the bone 12. One or more strands of operative suture 152 are passed through the soft tissue 10 to be reattached to bone 12. The operative suture strands 152 are then passed through the stay suture loop 142. When the operative suture 150 is arranged within the loop 142 of the stay suture 140, the loop 142 can be tensioned to trap the operative suture 150 between the suture seating surface 416 and the stay suture loop 142 as illustrated in FIG. 9C.

The inserter shaft 222 is then disposed in the previously drilled bone hole 14 and the operative suture 150 is tensioned to the desired degree by pulling on the free ends 154, as illustrated in FIG. 9C. The suture anchor 102 is inserted into the bone hole 14 by rotating the inserter shaft 222, as illustrated in FIGS. 9C and 9D, causing the suture anchor to advance further into the bone and to be in proximity to the suture seating member 412. While the suture anchor is rotated and advanced into bone, the free suture limbs 154 of the operative suture are constrained by the distal-facing suture seating surface 416 formed on the suture seating member 412, which does not rotate, thus maintaining the position of the operative suture strands.

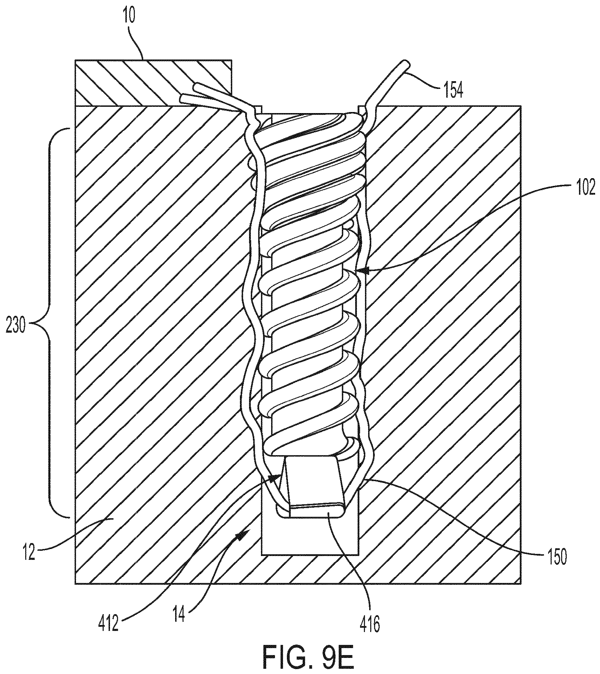

Once the anchor construct 230 is properly seated, the stay suture 140 is removed and pulled out of the lumen 228, as illustrated in FIG. 9E. Additionally, the inserter shaft 222 is removed, leaving the anchor construct 230, formed from the suture anchor 102 and the suture seating member 412, within the bone hole 14. A person skilled in the art will understand that the positioning of the operative suture 150 between the walls of the bone hole and the anchor construct 230 maintains sufficient tension on the operative suture, and the suture seating surface described herein reduce the chances of suture overlap.

The methods and systems described herein can have different variations. For example, multiple sutures can be used to couple tissue to bone. Also, one or more operative sutures can be loaded within the anchor before or during a surgical procedure. For example, a suture anchor can have at least one operative suture pre-loaded thereto such that the suture anchor includes the suture. Furthermore, the anchor may be pre-loaded on the inserter shaft.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device, e.g., the shafts, can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the components of the system described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred the components are sterilized. This can be done by any number of ways known to those skilled in the art including beta or gamma radiation, ethylene oxide, steam, and a liquid bath (e.g., cold soak).

One skilled in the art will appreciate further features and advantages of the described subject matter. Accordingly, the present disclosure is not to be limited by what has been particularly shown and described, except as indicated by the appended claims.

### Numbered aspects of the invention:

1. A suture anchor system, comprising:
   a cannulated inserter tool having a lumen extending therethrough and an elongate inserter shaft with an anchor receiving portion that is proximal to a distal extension, wherein the anchor receiving portion has at least one drive feature formed thereon and wherein the distal extension has a diameter that is less than a diameter of the anchor receiving portion;
   a cannulated suture anchor removably disposed on the anchor receiving portion of the inserter tool, and at least one bone-engaging feature disposed on an external surface thereof; and
   a cannulated suture seating member removably disposed on the distal extension in a clearance fit, the suture seating member being rotationally independent of the suture anchor and having a lumen extending therethrough with an opening on a distal end thereof, wherein the distal portion of the suture seating member has a suture seating surface.
2. The suture anchor system of aspect 1, further comprising at least one stay suture loop extending through the lumen and the opening of the suture seating member.
3. The suture anchor system of aspect 1, wherein the suture seating surface is at least one of a flat surface and a contoured surface.
4. The suture anchor system of aspect 3, wherein the contoured surface is a generally concave surface.
5. The suture anchor system of aspect 1, wherein the suture seating member further includes a collapsible section, and the suture seating surface is arranged on the distal end of the collapsible section.
6. The suture anchor system of aspect 1, wherein the suture seating member includes a pair of opposed suture seating features arranged on opposed side surfaces of the suture seating member, the suture seating features being configured to maintain a desired orientation of one or more operative suture strands.
7. The suture anchor system of aspect 1, wherein a length of the anchor receiving portion is greater than or equal to a length of the suture anchor.
8. The suture anchor system of aspect 1, wherein the suture seating member is spaced a distance from the suture anchor along the inserter shaft.
9. The suture anchor system of aspect 1, wherein the anchor receiving portion has a non-cylindrical cross-sectional shape.
10. The suture anchor system of aspect 9, wherein the anchor receiving portion has a hexagonal cross-sectional shape.
11. The suture anchor system of aspect 1, wherein the suture seating surface is configured to maintain one or more operative sutures in a predetermined orientation.
12. The suture anchor system of aspect 1, wherein a distal end of the anchor receiving portion includes a shoulder that is adjacent to a proximal end of the suture seating member.
13. The suture anchor system of aspect 1, wherein the suture anchor includes at least one driven feature disposed in a lumen thereof that is configured to engage the drive member of the anchor receiving portion.
14. The suture anchor system of aspect 13, wherein the lumen of the suture anchor has a cross-sectional shape that is complementary to a cross-sectional shape of the anchor receiving portion.
15. A method for attaching soft tissue to bone, comprising:
   securing one or more operative sutures to a soft tissue to be reattached to bone;
   passing free suture limbs of one or more operative sutures through at least one stay suture loop formed from at least one stay suture, the at least one stay suture loop extending from an opening on a distal end of a cannulated suture seating member, the suture seating member being positioned distally to a suture anchor removably mounted on a cannulated inserter ;
   tensioning the at least one stay suture to close the at least one stay suture loop such that the one or more operative sutures are trapped between a suture seating surface arranged on the suture seating member and the at least one stay suture loop;
   placing the one or more operative sutures within a bone hole adjacent the soft tissue to be reattached to bone by inserting into the hole the inserter having the anchor and suture seating member mounted thereon;
   applying a desired amount of tension to the free suture limbs while the one or more operative sutures are positioned within the hole;
   implanting the suture anchor into the bone hole to engage bone while maintaining the desired tension on the one or more operative sutures to bring the soft tissue into a desired engagement with bone while maintaining a rotational position of the suture seating member and substantially maintaining a position of the free suture limbs of the one or more operative sutures relative to the suture seating member; and
   removing the at least one stay suture and inserter.
16. The method of aspect 15, wherein the cannulated suture anchor includes at least one bone-engaging feature disposed on an external surface thereof.
17. The method of aspect 15, wherein the at least one bone-engaging feature is one of a thread and bone engaging barbs.
18. The method of aspect 15, wherein implanting the suture anchor comprises rotating the suture anchor.
19. The method of aspect 18, wherein the suture seating member is not rotated when the suture anchor is rotated.
20. The method of aspect 15, wherein following removal of the inserter and the at least one stay suture, the one or more operative sutures are knotlessly secured between the suture anchor and the bone hole.
21. The method of aspect 15, wherein following removal of the inserter and the at least one stay suture, the suture anchor compresses the suture seating member against a bottom of the bone hole and the suture seating member compresses against the bottom of the bone hole a portion of the one or more operative sutures distal to the suture seating member.

## Claims

1. A suture anchor system, comprising:
a cannulated inserter tool (121) having a lumen extending therethrough and an elongate inserter shaft (122) with an anchor receiving portion (125) that is proximal to a distal extension (123), wherein the anchor receiving portion has at least one drive feature (126) formed thereon and wherein the distal extension has a diameter that is less than a diameter of the anchor receiving portion;
a cannulated suture anchor (102) removably disposed on the anchor receiving portion of the inserter tool, and at least one bone-engaging feature (106) disposed on an external surface thereof; and
a cannulated suture seating member (112) removably disposed on the distal extension in a clearance fit, the suture seating member being rotationally independent of the suture anchor and having a lumen (114) extending therethrough with an opening on a distal end (112d) thereof, wherein the distal portion of the suture seating member has a suture seating surface (116).

2. The suture anchor system of claim 1, further comprising at least one stay suture loop (142) extending through the lumen (114) and the opening of the suture seating member (112).

3. The suture anchor system of claim 1 or 2, wherein the suture seating surface (116) is at least one of a flat surface and a contoured surface, preferably, wherein the contoured surface is a generally concave surface.

4. The suture anchor system of any preceding claim, wherein the suture seating member (112) further includes a collapsible section (217), and the suture seating surface is arranged on the distal end of the collapsible section.

5. The suture anchor system of any preceding claim, wherein the suture seating member (112) includes a pair of opposed suture seating features arranged on opposed side surfaces of the suture seating member, the suture seating features being configured to maintain a desired orientation of one or more operative suture strands.

6. The suture anchor system of any preceding claim, wherein a length of the anchor receiving portion (125) is greater than or equal to a length of the suture anchor.

7. The suture anchor system of any preceding claim, wherein the suture seating member (112) is spaced a distance from the suture anchor (102) along the inserter shaft.

8. The suture anchor system of any preceding claim, wherein the anchor receiving portion (125) has a non-cylindrical cross-sectional shape, preferably, wherein the anchor receiving portion (125) has a hexagonal cross-sectional shape.

9. The suture anchor system of any preceding claim, wherein the suture seating surface is configured to maintain one or more operative sutures in a predetermined orientation.

10. The suture anchor system of any preceding claim, wherein a distal end of the anchor receiving portion includes a shoulder that is adjacent to a proximal end of the suture seating member.

11. The suture anchor system of any preceding claim, wherein the suture anchor (102) includes at least one driven feature (110) disposed in a lumen (108) thereof that is configured to engage the drive member (126) of the anchor receiving portion.

12. The suture anchor system of claim 11, wherein the lumen (108) of the suture anchor has a cross-sectional shape that is complementary to a cross-sectional shape of the anchor receiving portion.

13. The suture anchor system of claims 1 to 12, for use in a method of attaching soft tissue to bone.
